# EUROPÄISCHE PATENTSCHRIFT

(11) **EP 1 737 363 B1**
(45) Veröffentlichungstag und Bekanntmachung des Hinweises auf die Patenterteilung: **24.10.2007**
(21) Anmeldenummer: 05715067.4
(22) Anmeldetag: 09.03.2005
(51) Int. Cl.: A61B 17/54

(54) **HORNHAUTHOBEL**
CALLOUS PLANE
RAPE POUR CALLOSITES

(30) Priorität: 07.04.2004 DE 102004017102
(43) Veröffentlichungstag der Anmeldung: 03.01.2007
(73) Patentinhaber: Mozart AG, 42655 Solingen (DE)
(72) Erfinder: SCHLIPKÖTER, Michael, 42699 Solingen (DE); WAHL, Christopher, 42651 Solingen (DE)
(74) Vertreter: Lippert, Hans-Joachim
(86) Internationale Anmeldenummer: PCT/DE2005/000402
(87) Internationale Veröffentlichungsnummer: WO 2005/099600

(56) Entgegenhaltungen:
- EP-A- 0 323 796
- EP-A- 1 216 664
- DE-A1- 3 320 594
- FR-A- 376 901
- FR-A- 515 352
- FR-A- 2 064 677

## Beschreibung

Die Erfindung betrifft einen Hornhauthobel mit einem Klingenkopf und einem Handgriff, wobei der Hornhauthobel eine in Arbeitsrichtung zur Körperoberfläche des Benutzers gewandte Unterseite und eine der Unterseite abgewandte Oberseite aufweist sowie in dem Klingenkopf eine Halterung mit einem unteren Halterungsteil und einem oberen Halterungsteil vorgesehen ist, in welcher eine Klinge so halterbar ist, dass ihre zum Einsatz vorgesehene Schneide zum Abhobeln von Hornhaut an der Unterseite des Hornhauthobels angeordnet ist.

Hornhauthobel weisen eine rasiermesserartige, scharfe Klinge auf, die besonders geschützt sowie möglichst sicher, das heißt unter anderem form- und lagestabil in dem Hornhauthobel angeordnet sein sollte. Diese Klingen weisen üblicherweise zwei an gegenüberliegenden Seiten der Klinge angeordnete Schneiden auf. Ein üblicher Hornhauthobel der eingangs genannten Art weist zum Beispiel eine Halterung auf, in der die Klinge durch ein unteres Halterungsteil gegen ein oberes Halterungsteil befestigt wird, wodurch die Klinge von oben her abgedeckt wird. Das Unterteil bildet jedoch an der Unterseite einen Formsprung, an dem die Schneide mit ihren Ecken gefährlich hervorsteht.

Um die Klinge besser zu schützen, wird in EP 1 216 664 A2 vorgeschlagen, die Klinge in einer Halterung aus Kunststoff einzubetten. Halterung und Klinge bilden hierbei eine integrale Einheit, die an dem Hornhauthobel befestigt wird. Hierbei werden die Schneidenecken zwar abgedeckt, die Ersatzschneide bleibt jedoch, da die Einheit zum Einsatz der Ersatzschneide um 180° am Hornhauthobel gedreht werden muss, bis auf ihre Ecken zweckmäßigerweise frei und bildet ein entsprechendes Verletzungspotential. Nachteilig ist ferner das verfahrensaufwendige Einbetten der Klinge in eine exakte Kunststoffform, welches zudem die Problematik der Haftung zwischen dem Metallwerkstoff der Klinge und dem Kunststoff sowie des notwendig sicheren Haltens der extrem flachen, harten und scharfkantigen Klinge in dem Kunststoffbett mit sich bringt, sodass ein gefährliches vorzeitiges Lockern der Klinge nicht auszuschließen ist. Zudem kann sich der Sitz der Einheit auf dem Hornhauthobel durch Drehung der Einheit zur Nutzung der Ersatzschneide lockern.

Dokument EP 0 323 796 offenbart einen Hornhauthobel gemäß des Oberbegriffs des Anspruchs 1.

Der Erfindung liegt daher die Aufgabe zugrunde, einen Hornhauthobel der eingangs genannten Art bereitzustellen, der weniger aufwendig herstellbar sowie sicherer handhabbar ist.

Diese Aufgabe wird durch Bereitstellung eines Hornhauthobels der eingangs genannten Art gelöst, dessen unteres Halterungsteil mit dem Handgriff fest verbunden ist und eine nach oben weisende Aufnahme für die Klinge aufweist und dessen oberes Halterungsteil die Klinge in der Aufnahme mit Hilfe einer Befestigungseinrichtung festhält.

Hiermit wird eine konstruktiv wenig aufwendige Lösung vorgeschlagen, die an der Unterseite keine Zweiteiligkeit der Halterung vorsieht, so dass hiermit keine sich an der Unterseite ereignende Verschiebung oder Lockerung der Halterungsteile stattfinden kann. Ferner können hierdurch an der Unterseite Absätze oder andere Formsprünge vermieden werden, die sich nach dem Stand der Technik bei einer Verbindung des oberen Halterungsteils mit dem Handgriff durch das untere Halterungsteil bilden und die sich beim Abhobeln zum Beispiel an erhöhten Hautpartien gefährlich verhaken und somit zum gefahrvollen Springen des Hornhauthobels führen können. Ferner ist hierdurch die Klinge in den unteren Formverlauf integriert und ragt nicht gefährlich über diesen hinaus. Dies kann zudem zur Konzeption eines ansprechenden Designs genutzt werden.

Von daher ist es vorteilhaft, wenn in einer Weiterbildung die Unterseite des Klingenkopfes übergangslos von dem unteren Halterungsteil in die Unterseite des Handgriffs übergehen kann. Das untere Halterungsteil kann zudem einstückig mit dem Handgriff verbunden sein. Somit wird ein durchgehender Formverlauf vorgeschlagen, der die Handhabung des Hornhauthobels weiter erleichtert und damit sicherer macht. Hierdurch kann beispielsweise der Anstellwinkel, d.h. der Winkel, in dem die Klinge über die zu bearbeitende Körperfläche gleitet, gefahrlos verändert werden, ohne dass sich Hornhauterhöhungen in Formsprüngen an der Unterseite des Hornhauthobels verhaken oder festsetzen können, sodass der Hornhauthobel durch Losreißen von diesen Erhöhungen unvermittelt springen kann. Zudem kann hiermit eine besonders ansprechende, ästhetische Form mit einer durchgehenden Formlinie geschaffen werden, die die allgemeine Akzeptanz eines Hornhauthobels erhöht.

Das untere Halterungsteil kann in einer bevorzugten Weiterbildung die gesamte Unterseite der gehalterten Klinge bis auf den mittleren Abschnitt der zum Einsatz vorgesehenen Schneide abdecken. Hierdurch wird eine Verletzungsgefahr durch die Ersatzschneide von der Unterseite her vermieden, die gerade beim Zurückführen des Hornhauthobels in seine Ausgangsposition auftreten kann, indem der Hornhobel hierbei in der Regel in Schneidrichtung der Ersatzschneide geführt wird. Ferner kommt dies wiederum einem ansprechenden Design entgegen.

Der Klingenkopf kann von seiner Oberseite her die gehalterte Klinge bis auf einen mittleren Abschnitt ihrer Schneide im wesentlichen abdecken. Hierdurch ist eine gefahrlosere und leichtere Handhabung der Klinge von oben her möglich, wie zum Beispiel beim Befestigung der Klinge durch Andrücken des oberen Halterungsteils gegen das untere Halterungsteil. Ferner kann in einer weiteren Ausführung der Formverlauf der Oberseite übergangslos von dem Klingenteil in den Handgriff übergehen. Dies kann wiederum zur Steigerung des Designs des Hornhauthobels beitragen.

Die Unterseite des oberen Halterungsteils und die Oberseite des unteren Halterungsteils können eine etwa parallel zueinander verlaufende gebogene Form aufweisen, deren Biegeachse in Funktionsrichtung des Hornhauthobels verläuft, sodass die gehalterte Klinge unter Biegespannung zwischen den Halterungsteilen angeordnet ist. Durch die Biegung der Klinge wird die Formstabilität der Klinge erhöht. Ferner wirkt die gebogene Klinge zwischen den Halterungsteilen wie eine Blattfeder, die die Halterungsteile gegen die noch zu beschreibenden Befestigungsmittel drängt, sodass die Halterungsteile lagestabiler zueinander gehalten werden können.

Hierbei kann vorgesehen sein, dass, entsprechend üblicher Hornhauthobel, die Halterungsteile seitlich nach oben hin gebogen sind und die Funktionsrichtung in Längsachse des Hornhauthobels von dem Klingenkopf zum Handgriff hin und verläuft. Hierbei ist eine leichte Krafteinleitung über den Handgriff in die Klinge möglich. Die Funktionsrichtung kann jedoch auch parallel zur Unterseite und senkrecht zur Längsachse verlaufen, wodurch der Hornhauthobel seitlich geführt wird und somit ansonsten schwerer zugängliche Körperoberflächenreiche erreichen kann. Es kann ferner vorgesehen sein, dass die Halterung in verschiedene, bevorzugt in die beiden oben genannten Funktionsrichtungen drehbar ist und/oder die Klinge in die Aufnahme so einlegbar ist, dass sie mit ihrer Schneide in eine gewünschte Funktionsrichtung weist.

In einer Weiterbildung des erfindungsgemäßen Hornhauthobels weist das untere Halterungsteil eine Klingenöffnung auf, durch die die Schneide der Klinge so führbar ist, dass zumindest ein mittlerer Längsabschnitt der Schneide den Rand der Klingenöffnung an der Unterseite des unteren Halterungsteils um einen kleinen Betrag senkrecht zur Funktionsrichtung überragt. Hierbei kann der Umriss der Klingenöffnung beispielsweise einem Kreisbogenabschnitt entsprechen, dessen Radius in Funktionsrichtung verläuft. Durch diesen Betrag wird letztlich die maximale Dicke der abzuhobelnden Hornhautpartikeln bestimmt. Dieser Betrag kann einstellbar sein, indem die Klinge beispielsweise weiter durch die Klingenöffnung hinausschiebbar ist.

Es können ferner zusätzliche, seitlich angeordnete Abdeckungen zur seitlichen Abdeckung der seitlichen, schneidlosen Kanten und/oder der Ersatzschneide der Klinge vorgesehen sein.

Die Aufnahme kann in der Oberseite des unteren Halterungsteils als Mulde ausgebildet sein, wobei die Klingenöffnung zur Freilegung des mittleren Bereiches der zum Einsatz vorgesehenen Schneide an der Unterseite des unteren Halterungsteiles an dem Muldengrund angeordnet ist.

Bevorzugt kann der Biegeradius der Unterseite des unteren Halterungsteils zumindest in dem Bereich der Klingenöffnung geringer als der Biegeradius der Unterseite des oberen Halterungsteils ausgelegt sein. Hierdurch wird die gehalterte Klinge entsprechend stärker als die Unterseite des unteren Halterungsteils gebogen und ragt somit mittig stärker über die Klingenöffnung hinaus. Ferner kann auch die Unterseite des Unterteils ein wenig in Längsrichtung gebogen, sodass eine leicht konvex gewölbte Unterseite entsteht. Durch diese gewölbte Unterseite schmiegt sich der Hornhauthobel leichter und damit gefahrloser an die zu bearbeitende Körperpartie an. Ferner wird hierdurch der durch die Öffnung hinausragende Längsabschnitt der Schneide von unten her leichter zugänglich und muss somit zum Abhobeln von Hornhaut weniger weit durch die Öffnung hinausragen.

Bevorzugt weist die Befestigungseinrichtung eine Führungsvorrichtung zum geführten Einlegen der Klingen in die Aufnahme sowie zum Ineinanderführen beider Halterungsteile auf. Diese Führungsvorrichtung kann zum Beispiel Führungssockel aufweisen, die an den Ecken der Aufnahme angeordnet sind und die sich nach oben hin erstrecken, wobei die gehalterte Klinge sowie das obere Halterungsteil seitlich an den Führungssockeln anliegen. Hierdurch wird die gehalterte Klinge von mehreren Seiten gehalten, sodass ein Andrücken der Klinge gegen das untere Halterungsteil, insbesondere unter gleichzeitigem Aufbau einer Biegespannung der Klinge, gefahrlos erfolgen kann, da die Klinge seitlich nicht aus der Aufnahme herausrutschen kann. Die Seiten der Führungssockel, an denen die gehalterte Klinge anliegt, können in einer Weiterbildung pyramidal ausgebildet sein, wodurch das Hineinführen der Klinge in die Aufnahme erleichtert wird.

Um ein gefahrloses Einlegen der Klinge in die Aufnahme zu ermöglichen, ist in einer Weiterbildung der Erfindung an den beiden Längsseiten der Aufnahme jeweils eine Einlegemulde zum Einlegen der mit zwei Fingern erfassten Klinge in die Aufnahme vorgesehen. Diese Einlegemulden werden zweckmäßigerweise durch das obere Halterungsteil verdeckt, wodurch der Benutzer von einem eventuellen Überstand der Klinge geschützt wird.

In einer vorteilhaften Weiterbildung sind die beiden Halterungsteile auf einer Seite durch eine Steckverbindung verbindbar und auf der gegenüberliegenden Seite verrastbar. Hierbei kann die Steckverbindung an dem oberen Halterungsteil zwei parallel zueinander beabstandete, sich in Funktionsrichtung erstreckende Steckvorsprünge aufweisen, die unter zwei entsprechend von dem Aufnahmegrund und zueinander beabstandete Haltevorsprünge gegen einen Anschlag führbar sind, wobei die zueinander gewandten Seitenflächen der Vorsprünge nach dem Prinzip der schiefen Ebene aneinander abgleiten. Hierbei ist, nachdem die Klinge in der Aufnahme gelagert ist, das obere Halterungsteil mit seinen Steckvorsprüngen unter die Haltevorsprünge des unteren Halterungsteils einsteckbar und in einer Schwenkbewegung durch Zusammendrücken beider Halterungsteile mit dem unteren Halterungsteil verrastbar. Durch diese Schwenkbewegung gleiten die zueinander gewandten Seitenflächen der Vorsprünge voneinander ab, welches durch Abschrägung dieser Seitenflächen erleichtert wird. Denkbar sind auch andere Ausbildungsformen der Seitenflächen, wie zum Beispiel eine konvexe Seitenfläche des Haltevorsprunges, die an einer entsprechend konkav ausgebildeten Seitenfläche des Steckvorsprunges abgleitet. Anstatt einer Steckverbindung kann das obere Halterungsteil über ein Gelenk verschwenkbar an dem unteren Halterungsteil befestigt sein. Zweckmäßigerweise ist die Klinge zum Einlegen in die Aufnahme mit einem Abschnitt unter die Haltevorsprünge schiebbar. Hierdurch ist die Klinge in der Aufnahme fixiert und kann gefahrloser durch das obere Halterungsteil gegen das untere Halterungsteil gepresst werden.

In einer anderen Ausbildung der Befestigungseinrichtung kann eine Nut/Feder-Führung vorgesehen sein, durch die, wie weiter unten näher erläutert, das obere Halterungsteil bevorzugt in Funktionsrichtung über das untere Halterungsteil führbar ist. Hierbei kann die Nut/Feder-Führung eine Führungsschiene und eine Führungsnut mit einem beispielsweise schwalbenschwanzartigen Querschnitt aufweisen, wodurch ein festerer Sitz der Führungsschiene in der Führungsnut senkrecht zur Längsrichtung erzielt sowie die Gefahr eines Aufweitens des oberen Halterungsteiles infolge der Anpresskraft der Klinge minimiert wird. Der Querschnitt der Führungsform kann in einer anderen Ausführung eine einseitige Schwalbenschwanzform mit in etwa parallel zueinander und in einem Winkel ungleich 90° zur Nutöffnung verlaufenden Nutinnenseitenflächen aufweisen, durch die somit lediglich eine Hinterschneidung gebildet wird. Diese Hinterschneidung kann hierbei an der unteren Nutinnenseitenfläche angeordnet sein. Die Nut/Feder-Führung kann ferner an seitlichen Innenflächen des unteren Halteteil, wodurch das obere Halteteil entsprechend innen in der bzw. über der Aufnahme für die Klinge angeordnet ist, und/oder an seitlichen Außenflächen des unteren Halteteil vorgesehen sein. Hierdurch kann das obere Halterungsteil zum Einkoppeln in die Nut/Feder-Führung das untere Halteteil von oben übergreifen und somit die Aufnahme und damit die gehalterte Klinge von oben her vollständig abdecken.

In einer weiteren Ausbildung der Befestigungseinrichtung wird eine Kombination mit den oben beschriebenen Befestigungsarten, d.h. bevorzugt mit der Nut/Feder-Führung und der Steckverbindung, vorgeschlagen. Selbstverständlich fallen auch andere Befestigungsarten, die die Klinge durch das obere Halterungsteil in der Aufnahme in geeigneter Weise festhalten, in den Rahmen der Erfindung.

Die Befestigungseinrichtung kann ferner Verrastungsmittel aufweisen, durch die das obere Halterungsteils und das untere Halterungsteil miteinander verrastet werden können. Bevorzugt soll das obere Halterungsteil durch Gegendrücken gegen das untere Halterungsteil ohne weiteres Werkzeug durch die Verrastungsmittel verrastet werden können. Hierzu kann das eine Halterungsteil mindestens einen Rasthaken gemäß dem Stand der Technik aufweisen. Der Rasthaken kann hierbei seitlich an einem der Halterungsteile angeordnet sein und zur Verrastung das andere Halterungsteil seitlich umgreifen, wobei sein freies Ende mit dem querlaufenden Hakenendabschnitt unter Biegespannung an der von dem einen Halterungsteil abgewandten Seite des anderen Halterungsteils anliegt. Hierbei kann an dieser Stelle eine Aussparung vorgesehen sein, in die der querlaufende Endhakenabschnitt zumindest so eingreift, dass er nicht über die Unterseite des Klingenkopfes ragt. Der Rasthaken kann ferner an seinem Ende eine weitere, bevorzugt rechtwinklig zum Hakenende und senkrecht zur Funktionsrichtung verlaufende Erstreckung aufweisen, mit der er in eine in dem anderen Halterungsteil vorgesehene Öffnung oder dergleichen eingreift, wodurch eine form- und kraftschlüssige Verbindung zwischen den beiden Halterungsteilen erzielt werden kann. In einer anderen Ausführungsform kann der Rasthaken seitliche Erstreckungen aufweisen, die nach Überwindung eines an dem anderen Halterungsteil angeordneten Vorsprunges oder dergleichen durch elastisches Aufbiegen des Rasthaken in vorgesehene seitliche Ausnehmungen wie Taschen oder dergleichen des anderen Halterungsteiles eingreifen können.

An der Oberseite des oberen Halterungsteils kann eine Andrückmulde zum Andrücken des oberen Halterungsteils gegen das untere Halterungsteil vorgesehen sein, um einen sicheren Halt eines Druck ausübenden Fingers des Benutzers zu gewährleisten. Diese Andrückmulde kann ferner zum Andrücken des Hornhauthobels gegen eine Unterlage durch den Finger des Benutzers dienen, wodurch eine leichte und dosierbare Druckbeaufschlagung des Hornhauthobels gegen die Unterlage möglich ist. Bevorzugt ist diese Andrückmulde ergonomisch ausgebildet. Ferner kann die Andrückmulde an dem freien Ende des Klingenkopfes und damit entfernt von der zum Einsatz vorgesehenen Schneide angeordnet sein. Ferner können die Verrastungsmittel, insbesondere der Rasthaken, so ausgebildet und so angeordnet sein, dass sie über Betätigung der Andrückmulde entrastbar sind.

In einer Weiterbildung ist in dem oberen Halterungsteil ein Durchgang zum Sammeln und Auswerfen abgehobelter Hornhautpartikel vorgesehen, wobei der Durchgang in Arbeitslage über der Klingenöffnung angeordnet ist. Zweckmäßigerweise ist der Durchgang zur Oberseite des oberen Halterungsteils hin konisch erweitert, wobei die den freien Ende des Klingenkopfes zugewandte Innenwand des Durchganges eine Gleitschräge aufweist, über die die gehobelten Hornhautpartikel entgegen der Funktionsrichtung abgleiten können. Hierdurch wird vermieden, dass sich Hornhautpartikel zwischen Klinge und Halterungsteil schieben und somit die Klinge blockieren können.

Der Handgriff des Hornhauthobels kann an seinem freien Ende ein Griffteil und an seinem zum Klingenkopf gewandten Ende ein Anschlussteil aufweisen. Hierbei kann in dem Anschlussabschnitt eine von der Oberseite zur Unterseite führende Sichtöffnung zur Sicht auf die zu bearbeitende Körperoberfläche eines Benutzers eingelassen sein. Die Sichtöffnung kann seitlich durch zwei gabelförmig angeordnete Stege begrenzt sein. Die Sichtöffnung kann ferner nach oben hin konisch erweitert sein, um hierdurch einen möglichst weiten Blick auf die zu bearbeitende Oberfläche zu ermöglichen. In der Sichtöffnung kann eine zumindest teilweise die Sichtöffnung ausfüllende Lupe zur optischen Vergrößerung des Ausschnittes auf die zu bearbeitende Oberfläche vorgesehen sein, wodurch ein exaktes und sicheres Arbeiten erleichtert wird.

Das Griffteil kann ergonomisch der Hand des Benutzers angepasst sein. Hierzu weist das Griffteil in einer möglichen Ausbildung im wesentlichen zwei Abschnitte, einen sich zum freien Ende erstreckenden Handballenabschnitt und einen an das Anschlussteil angrenzenden Fingerabschnitt, auf. Hierbei können in dem Handballenabschnitt seitliche konvexe Griffpolster zur Anpassung an die Handinnenfläche des Benutzers und/oder in dem Fingerabschnitt Fingermulden vorgesehen sein.

Zweckmäßigerweise kann die Oberfläche des Griffteils zumindest teilweise aus rutschhemmendem und/oder nachgiebigem Material gefertigt sein. Denkbar sind auch Beschichtungen mit einem rutschhemmenden Werkstoff. Hierdurch liegt das Griffteil besonders sicher in der Hand des Benutzers an.

Bevorzugt kann der Fingerabschnitt umfänglich mehrere Fingermulden aufweisen. Hierbei können beispielsweise bei einer rechteckigen Querschnittsform des Fingerabschnittes an allen vier Außenflächen Fingermulden vorgesehen sein. Hierdurch kann der Hornhauthobel bei einer Drehung in der Hand in mindestens vier Positionen sicher gehalten werden, sodass der Hornhauthobel mit verschiedenen Griffpositionen in der Hand sicher über die Hautoberfläche geführt werden kann. Dies ist insbesondere dann von Vorteil, wenn eine Unterseite einer Körperpartie mit dem Hornhauthobel bearbeitet werden soll, sodass die Unterseite des Hornhauthobels mit der Klingenschneide in Schwerkraftrichtung nach oben weist.

Der Hornhauthobel kann aus Kunststoff gefertigt sein. Bevorzugt kann der Hornhauthobel als Spritzgussteil hergestellt sein. Hierbei kann das untere Halterungsteil einstückig mit dem Handgriff verbunden sein. Das obere Halterungsteil kann zudem beispielsweise über ein Foliengelenk einstückig mit dem unteren Halterungsteil verbunden sein.

Die vorliegende Erfindung wird anhand dreier Ausführungsbeispiele und einer zugehörigen Zeichnung näher erläutert. In der Zeichnung zeigen:
- Fig. 1: eine perspektivische Aufsicht auf ein erstes Ausführungsbeispiel eines Hornhauthobels mit einem oberen Halterungsteil,
- Fig. 2: eine perspektivische Aufsicht auf den Hornhauthobel gemäß Fig. 1 ohne oberes Halterungsteil und mit eingelegter Klinge,
- Fig. 3: eine Unteransicht des Hornhauthobels gemäß Fig. 1 mit Klinge,
- Fig. 4: eine perspektivische Aufsicht auf das obere Halterungsteiles des Hornhauthobels gemäß Fig. 1,
- Fig. 5: schematisch eine Querschnittsfläche gemäß der in Fig. 3 durch den Verlauf A-A gekennzeichneten Stelle,
- Fig. 6: perspektivische Aufsicht auf ein zweites Ausführungsbeispiel des erfindungsgemäßen Hornhauthobels ohne oberes Halterungsteil und mit eingelegter Klinge,
- Fig. 7: perspektivische Aufsicht auf das obere Halterungsteil des Hornhauthobels gemäß Fig. 6,
- Fig. 8: schematisch eine Querschnittsfläche des Hornhauthobels gemäß Fig. 6 ohne oberes Halterungsteil und mit eingelegter Klinge im freien Endbereich
- Fig. 9: schematisch eine Querschnittsfläche wie in Fig. 8, jedoch mit eingeschobenem oberen Halterungsteil,
- Fig. 10: eine schematische mittlere Längsschnittfläche des freien Endes des Klingenkopfs,
- Fig. 11: eine perspektivische Aufsicht auf ein drittes Ausführungsbeispiel des erfindungsgemäßen Hornhauthobels mit einem oberen Halterungsteil,
- Fig. 12: eine perspektivische Aufsicht auf den Hornhauthobel gemäß Fig. 11 ohne oberes Halterungsteil und mit eingelegter Klinge,
- Fig. 13: eine perspektivische Unteransicht des Hornhauthobels gemäß Fig. 11 ohne oberes Halterungsteil,
- Fig. 14: eine perspektivische Aufsicht auf das obere Halterungsteiles des Hornhauthobels gemäß Fig. 11 und
- Fig. 15: eine perspektivische Untersicht des oberen Halterungsteiles des Hornhauthobels gemäß Fig. 11.

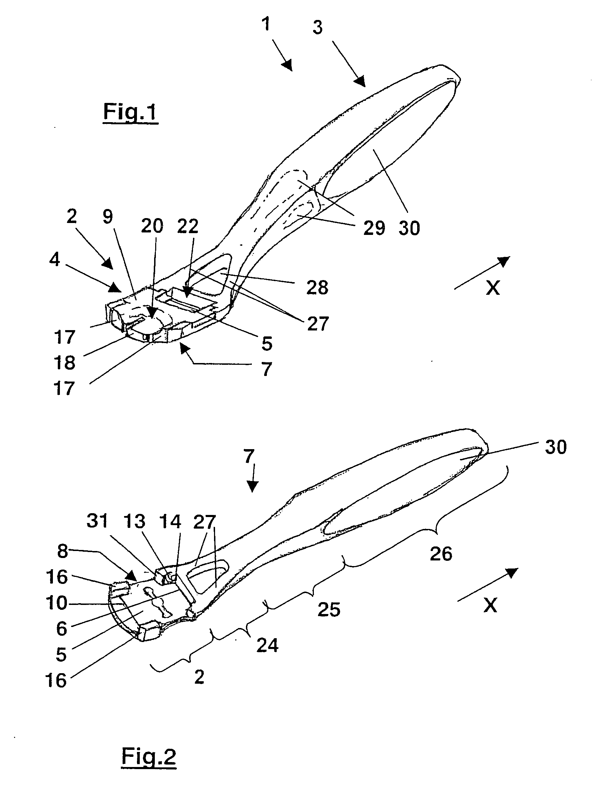

Anhand der Figuren 1 bis 5 wird ein erstes Ausführungsbeispiel eines erfindungsgemäßen Hornhauthobels 1 mit eingezeichneter Funktionsrichtung X erläutert. Fig. 1 zeigt den Hornhauthobel 1 mit einem Klingenkopf 2 und einem Handgriff 3 in einer perspektivischen Aufsicht, wobei der Hornhauthobel 1 eine in Arbeitslage zur zu behandelnden Hautoberfläche eines Benutzers gewandte Unterseite und eine Oberseite aufweist. In dem Klingenkopf 2 ist eine Halterung 4 vorgesehen, in der eine Klinge 5 so halterbar ist, dass ihre in dieser Abbildung kaum sichtbare, da verdeckte Schneide 6 zum Abhobeln von hier nicht dargestellter Hornhaut an der Unterseite des Hornhauthobels 1 angeordnet ist. Die Halterung 4 weist ein unteres Halterungsteil 7 mit einer nach oben weisenden Aufnahme 8 für die Klinge 5 und ein oberes Halterungsteil 9 zum Anpressen und Befestigen der Klinge 5 durch eine Befestigungseinrichtung in der Aufnahme 8 auf. Das untere Halterungsteil 7 ist hierbei einstückig mit dem Griff 3 verbunden. Die Aufnahme 8 ist zwar in Fig. 1 durch das obere Halterungsteil 9 verdeckt, jedoch in Fig. 2 unverdeckt gezeigt. Die Klinge 5 wird von dem oberen Halterungsteil 9 bis auf einen mittleren Bereich der Schneide 6 im wesentlichen abgedeckt.

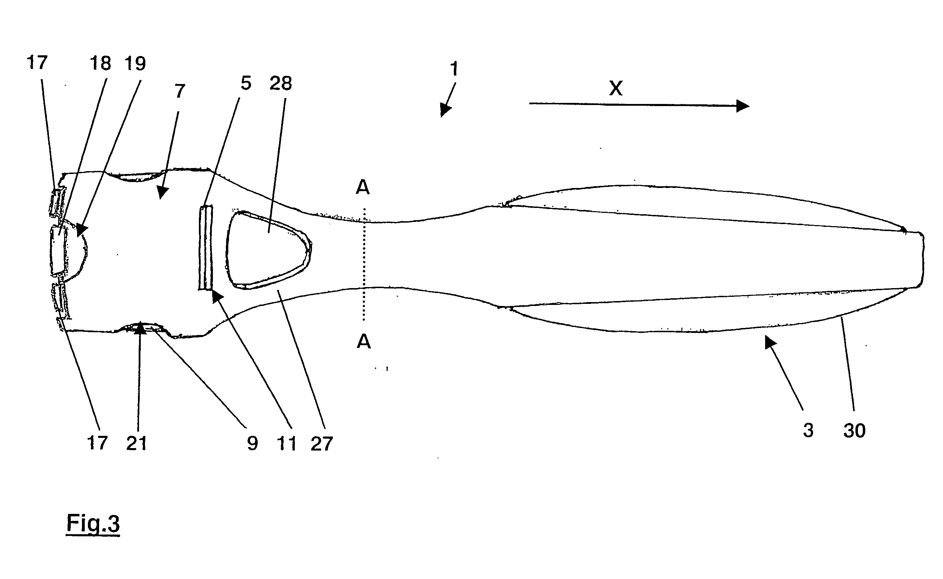

In Fig. 2 ist in perspektivischer Aufsicht der Hornhauthobel 1 ohne oberes Halterungsteil und mit in der Aufnahme 8 angeordneter Klinge 5 dargestellt. Hierbei wird eine übliche Klinge 5 mit zwei Schneiden 6, 10 gezeigt, wobei in der hier dargestellten Position die Schneide 6 zum Einsatz kommt, während die andere als Ersatzschneide 10 von dem oberen Halterungsteil 9 und, wie anhand von Figur 3 noch näher erläutern wird, von dem unteren 7 Halterungsteil abgedeckt wird. Selbstverständlich können auch andere Klingenformen verwandt werden, für die eventuell für einen mit dieser Lehre vertrauter Fachmann offensichtliche Abänderungen der Halterungsteile notwendig sein würden.

In Fig. 3 ist der Hornhauthobel 1 in einer Unteransicht gezeigt. Hierbei geht die Unterseite des unteren Halterungsteils 7 übergangslos in die Unterseite des Handgriffs 3 über. Ferner deckt der Klingenkopf 2 von seiner Unterseite her die gehalterte Klinge 5 bis auf einen mittleren Abschnitt ihrer Schneide 6 ab, wobei das untere Halterungsteil 7 eine Klingenöffnung 11 aufweist, durch die die Schneide 6 der Klinge 5 teilweise geführt ist. Die Unterseite des oberen Halterungsteils 9 und die obere Seite des unteren Halterungsteils 7 weisen eine etwa parallel zueinander verlaufende, gebogene Form auf, deren Biegeachse in etwa in Funktionsrichtung X des Hornhauthobels 1 verläuft. Hierdurch wird die zwischen den Halterungsteilen 7, 9 angeordnete Klinge 5 mit einer Biegespannung beaufschlagt, durch die die Klinge 5 gleich einer Blattfeder die beiden Halterungsteile 7, 9 gegen die noch weiter zu beschreibenden Befestigungsmittel auseinander drückt. Somit wird eine gesteigerte Lagestabilität der Halterungsteile 7, 9 und der darin angeordneten Klinge 5 erzielt.

Ferner sind die Unterseiten beider Halterungsteile 7, 9 gebogen, wobei, wie in den Zeichnungen angedeutet, der Biegeradius der Unterseite des unteren Halterungsteils 7 zumindest im Bereich der Klingenöffnung 11 geringer als der Biegeradius der Unterseite des oberen Halterungsteils 7 ist. Hierdurch weist die Klinge 5 eine stärker gebogene Biegelinie als der Bereich um die Klingenöffnung 11 auf, sodass die Klinge mittig weiter als zum Rand hin über die Klingenöffnung 11 hinausragt. Hierdurch ist ein zielgerichtetes und feines Abhobeln von Hornhautpartikeln möglich.

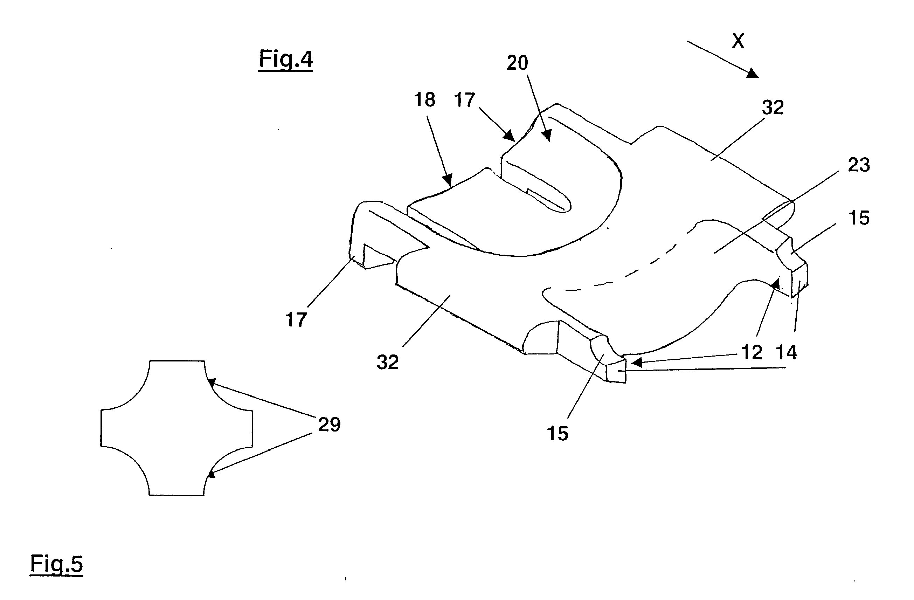

In Fig. 4 wird das obere Halterungsteil 9 in perspektivischer Aufsicht gezeigt. Hierbei weist das obere Halterungsteil 9 zwei sich in Funktionsrichtung X erstreckende Steckvorsprünge 12 auf, die unter zwei in Fig. 2 gezeigten Haltevorsprünge 13 jeweils gegen einen Anschlag 14 führbar sind, wobei die zueinander gewandten Seitenflächen der Vorsprünge 12, 13 nach dem Prinzip der schiefen Ebene aneinander abgleiten. Hierzu ist in dem hier gezeigten Ausführungsbeispiel die entsprechende Seitenfläche 15 der Steckvorsprünge 12 als konkave Seitenfläche 15 mit einem nach innen führenden Radius ausgeführt. Die Haltevorsprünge 13 weisen eine zu den Steckvorsprüngen 12 angepasste Beabstandung zu dem Aufnahmegrund auf. Wie ferner in Figur 2 ersichtlich, ist die eingelegte Klinge 5 mit den Ecken ihrer Schneide 6 unter die Haltevorsprünge 13 geführt. Hierdurch werden die Ecken abgedeckt. Ferner wird die Klinge 5 hierdurch lagestabil gehalten.

Ferner ist eine Führungsvorrichtung zum geführten Einlegen der Klinge 5 in die Aufnahme 8 sowie zum Ineinanderführen beider Halterungsteile 7, 9 vorgesehen. Hierbei weist die Führungsvorrichtung Führungssockel 16 auf, die an den Ecken der Aufnahme 8 angeordnet sind und sich nach oben hin erstrecken. Die gehalterte Klinge 5 liegt hierbei seitlich an den Führungssockeln 16 an, wodurch die Klinge 5 in Klingenebene form- und lagestabil gehalten wird. Ferner, wie in Fig. 1 gezeigt, liegt das obere Halterungsteil 9 mit sich seitlich erstreckenden Führungsvorsprüngen 32 ebenfalls seitlich an den Führungssockeln 16 an, sodass hierdurch ebenfalls eine verbesserte Lagestabilität der Klinge 5 erzielt wird.

Das obere Halterungsteil 9 weist an seinem den Führungssockeln 16 gegenüberliegenden Ende zwei Führungshaken 17 auf, die, wie in Fig. 1 ersichtlich, in Einbaulage stirnseitig über das freie Ende des unteren Halterungsteils 7 greifen, wodurch eine weitere Steigerung der Lagestabilität des oberen Halterungsteils 9 sowie der zwischen den Halterungsteilen 7, 9 gelagerten Klinge 5 erzielt wird. Ferner ist an dem oberen Halterungsteil 9 ein Rasthaken 18 vorgesehen, der zwischen und parallel beabstandet zu den beiden Führungshaken 17 gleich einer Blattfeder angeordnet ist und ebenfalls stirnseitig über das freie Ende des unteren Halterungsteils 7 übergreift. Hierbei greift der Rasthaken 18 so in eine Aussparung 19 der Unterseite des unteren Halterungsteils 7 ein, dass er unter Biegespannung an der Unterseite anliegt. Zudem ist vorgesehen, dass sich die Seitenflächen 31, mit denen die Führungssockel 16 in Halterungslage seitlich an der Klinge 5 sowie an dem oberen Halterungsteil 9 anliegen, pyramidal nach oben hin verjüngen, um hierdurch das Einlegen der Klinge 5 bzw. das Ineinandergreifen des oberen Halterungsteils 9 in das untere Halterungsteil 7 zu erleichtern.

An der Oberseite des oberen Halterungsteils 9 ist zudem eine Andrückmulde 20 zum Andrücken des Hornhauthobels 1 gegen eine hier nicht dargestellte Unterlage vorgesehen. Da diese Andrückmulde 20 in Einbaulage direkt über der Klinge 5 angeordnet ist, kann ein eingreifender Finger des Benutzers hier gezielt Druck auf den Klingenkopf 2 ausüben. Ferner können über diese Griffmulde 20 die beiden Klingenteile 7, 9 gefahrlos zusammengedrückt werden können, indem der Benutzer ober- und unterseitig an dem Klingenkopf 2 angreift.

Wie in Fig. 3 ersichtlich, weist das untere Halterungsteil 7 an seinen Längsseiten zwei Einlegaussparungen 21 auf, durch die das Einlegen der Klinge 5 in die Aufnahme 8 erleichtert wird. Die Einlegaussparungen 21 werden beim Zusammenfügen der beiden Halterungsteile 9, 10 durch das obere Halterungsteil 9 abgedeckt, sodass die eingelegte Klinge mit ihren schneidlosen Seitenkanten von oben ebenfalls vollständig abgedeckt ist.

Der Hornhauthobel 1 ist mit einem Durchgang 22 zum Sammeln und Auswerfen hier nicht dargestellter abgehobelter Hornhautpartikel versehen, wobei der Durchgang 22 in Arbeitslage über der Klingenöffnung 11 angeordnet ist. Dieser Durchgang 22 weist an seiner griffseitig abgewandten Innenwandung eine in Fig.4 deutlich erkennbare Abgleitschräge 23 auf, die entgegen der Funktionsrichtung X verläuft. Hierdurch wird das Auswerfen abgehobelter Hornhautpartikel, die über die Abgleitschräge 23 abgleiten, erleichtert und zugleich verhindert, dass diese Partikel die Klinge 5 gefährlich zusetzen.

Der Handgriff 3 des Hornhauthobels 1 weist, wie in Fig. 2 schematisch angedeutet, im wesentlichen drei Abschnitte auf, einen Anschlussabschnitt 24, einen Fingerabschnitt 25 und einen Handballenabschnitt 26. Hierbei ist in dem Anschlussabschnitt 24 eine dreieckige Sichtöffnung 28 zur Sicht auf die in Funktionsrichtung X nach der Schneide 6 liegende zu bearbeitende Oberfläche vorgesehen, die von zwei gabelförmigen Stege 27 seitlich begrenzt wird. Hierbei ist die Sichtöffnung 28 zweckmäßigerweise nach unten hin leicht konisch zulaufend ausgebildet.

Der Fingerabschnitt 25 und der Handballenabschnitt 26 bilden den eigentlichen Griffbereich des Handgriffs 3 und sind ergonomisch an die Hand des Benutzers angepasst. Hierzu weist in dieser Ausführungsform des Hornhauthobels 1 der Fingerabschnitt 25 Fingermulden 29 und der Handballenabschnitt 26 seitlich angeordnete, konvexe Griffpolster 30 auf. Diese Griffpolster 20 sind bevorzugt aus einem rutschhemmenden und/oder nachgiebigerem Material gefertigt. Denkbar ist auch eine rutschhemmende Beschichtung des Griffpolsters 20. Ebenfalls können Teile oder der vollständige Handgriff 3 mit einer rutschhemmenden Oberfläche versehen oder aus einem rutschhemmenden Material gefertigt sein.

Der Fingerabschnitt 25 weist eine im wesentlichen rechtwinklige Querschnittsform mit vier Seitenwänden mit abgerundeten Kanten auf, wobei in jeder der Seitenwände eine Fingermulde 29 vorgesehen ist, sodass sich insgesamt eine Querschnittsform ergibt, wie sie schematisch in Fig. 5 wiedergegeben ist. Hierdurch ist bei einer gedrehten Lage des Handgriffs 3 in der Hand des Benutzers weiterhin ein sicherer und ergonomisch angepasster Sitz des Handgriffs 3 in der Hand des Benutzers möglich, sodass der Hornhauthobel 1 griffsicher in verschiedensten Lagen in der Hand sicher geführt werden kann.

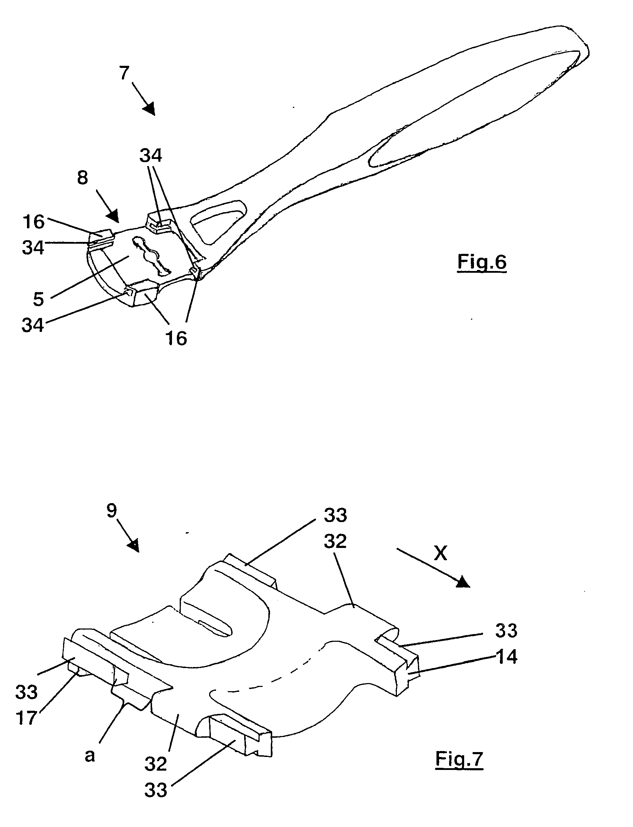

In den Figuren 6 bis 10 wird ein zweites Ausführungsbeispiel des Hornhauthobels 1 erläutert. Abweichend von dem zuvor beschriebenen Beispiel weist der Hornhauthobel 1 hier eine anderen Befestigungsart zum Befestigen des oberen Halterungsteiles 9 auf das untere Halterungsteil 7 auf. Hierzu sind, wie in der perspektivischen Aufsicht auf das obere Halterungsteil 9 in Figur 7 ersichtlich, im oberen Halterungsteil 9 seitliche Führungsschienen 33 vorgesehen, die sich in Längsrichtung erstrecken.

In Fig. 6, einer perspektivischen Aufsicht auf das untere Halterungsteil 7 mit eingelegter Klinge 5, ist ersichtlich, dass die Führungssockel 16 den Führungsschienen 33 angepasste Führungsnuten 34 zur Führung des oberen Halterungsteiles 9 beim Zusammenbringen beider Halterungsteile 7,9 aufweisen, wobei das obere Halterungsteil 9 in Funktionsrichtung X über das untere Halterungsteil 7 schiebbar ist. Die Führungsnuten 34 verlaufen dementsprechend an der Innenseite der Führungssockel 16 und in Längsrichtung des unteren Halterungsteiles 7. Um die eingelegte Klinge 5 mit dem Zusammenfügen der beiden Halterungsteile 7,9 wie vorgesehen durchzubiegen, ist es zweckmäßig, dass das obere Halterungsteil 9 zum Durchbiegen der Klinge 5 in einem ersten Schritt von oben her, d.h. senkrecht zur Klingenebene, gegen die Klinge 5 führbar und in einem weiteren Schritt durch Ineinandergreifen in Funktionsrichtung X von Führungsschienen 33 und -Nuten 34 mit dem unteren Halterungsteil 7 verbindbar ist. Hierzu sind zwischen den Führungsschienen 33, die in Einbaulage dem freien Ende des Hornhauthobels 1 zugewandt sind, und den Führungsvorsprüngen 32 Zwischenräume a vorgesehen, durch die die endseitigen Führungssockel 16 senkrecht zur Klingenebene führbar sind.

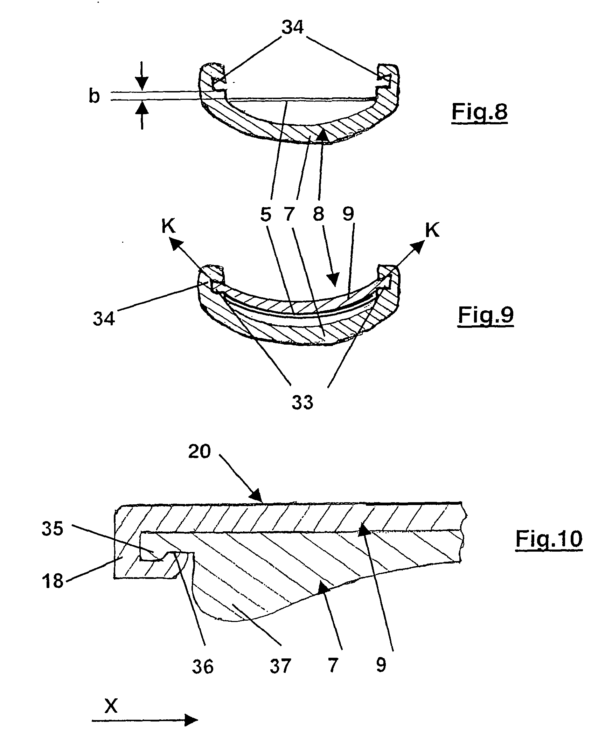

In den Figuren 8 bzw. 9 wird jeweils schematisch ein Querschnittsfläche durch den Klingenkopf 2 in etwa in Höhe der endseitigen Führungssockel 16 ohne bzw. mit dem in die Aufnahme 8 eingeschobenen oberen Halterungsteil 9 gezeigt. Hiermit soll die Lage der Klinge 5 in der Aufnahme 8 zwischen den Halterungsteilen 7, 9 demonstriert werden. In Fig. 8 liegt die Klinge 5 in der Aufnahme 8 nach oben hin lose auf, wobei zwischen den Führungsnuten 34 und der Oberseite der eingelegten Klinge 5 ein Abstand b vorgesehen ist, um ein seitliches Hineinrutschen der Klinge 5 in die Führungsnuten 34 zu verhindern. In Fig. 9 ist das obere Halterungsteil 9 mit seinen Führungsschienen 33 in die Führungsnuten 34 des unteren Halterungsteiles 7 hineingeschoben und drückt die Klinge 5 aufgrund seiner nach unten gewölbten Form nach unten, sodass die Klinge 5 unter Spannung zumindest in einem mittleren Bereich an der Unterseite des oberen Halterungsteiles 9 anliegt.

Die in den Figuren 8 und 9 gezeigten Darstellungen sollen lediglich das Prinzip verdeutlichen, nach dem die Klinge 5 in der Halterung 4 gehaltert ist. Somit fallen auch andere Ausführungsformen, die dieses Prinzip verwirklichen, in den Bereich dieser Erfindung.

Die Führungsschienen 33 bzw. die Führungsnuten 34 weisen in dem zweiten Ausführungsbeispiel einen schwalbenschwanzförmigen Querschnitt auf. Hierdurch wird die Kraft, die durch die nach unten gebogene Klinge 5 auf das obere Halterungsteil 9 übertragen wird, in etwa gemäß der gebogenen Oberseite des oberen Halterungsteiles 9 in Kraftrichtung K schräg in das untere Halterungsteil 7 eingeleitet. Hiermit wird, im Vergleich zu einem horizontalen Einleiten einer durch die Spannung der Klinge 5 erzeugten Kraft, die Gefahr eines möglichen Aufweitens des unteren Halterungsteiles 7 zumindest erheblich vermindert.

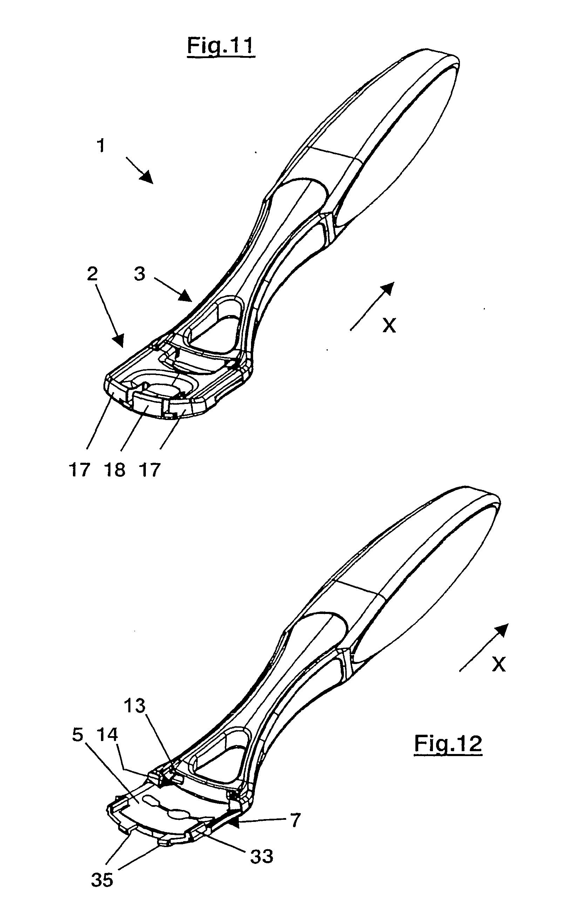

In Fig. 10 wird schematisch eine Längsschnittfläche in der Mittelachse des freien Endes des Klingenkopfes 2 mit dem über das Ende des unteren Halterungsteiles 7 ragenden Rasthaken 18 gezeigt. Abweichend von dem ersten Ausführungsbeispiel greift der Rasthaken 18 hier um einen endseitig an dem unteren Halterungsteil 7 vorgesehenen Rastvorsprung 35 in eine Rastnut 36 ein und liegt vorzugsweise unter Spannung an dem Rastvorsprung 35 und/oder in der Rastnut 36 an. Hierdurch wird vermieden, dass sich bei einer Relativbewegung des oberen Halterungsteiles 9 zum unteren Halterungsteil 7 entgegen der Funktionsrichtung X der Rasthaken 18 vorzeitig von dem Rastvorsprung 35 löst, sodass die beiden Halterungsteile 7, 9 im Klingenkopf 2 sicher fixiert sind. Diese Fixierung wird zusätzlich durch die Reibungskräfte, die zwischen Führungsschiene 33 und Führungsnut 34 auftreten, und durch die unter Spannung gehaltene Klinge 5 verstärkt.

Zum Schutz gegen ein vorzeitiges Abstreifen des Rasthaken 18 von dem Rastvorsprung 35 ist in Funktionsrichtung X eine Verdickung 37 vorgesehen, die vorzugsweise übergangslos in Funktionsrichtung X in die übrige Unterseite des unteren Halterungsteiles 7 übergeht.

Die Verdickung kann sich, wie aber hier nicht dargestellt, unterseitig weiter entgegen der Funktionsrichtung über den Rasthaken erstreckt, sodass der Rasthaken damit in eine Öffnung oder Nut eingreift. Zum leichteren Lösen des Rasthaken kann der Rasthaken ferner den Rastvorsprung so mit Spiel umgreifen, dass der Rasthaken durch Druck auf das obere Halterungsteil senkrecht zur Funktionsrichtung aus der Rastnut herausgehoben und entgegen der Funktionsrichtung über den Rastvorsprung hinausgeführt werden kann. Selbstverständlich kann das erste sowie das nun folgende dritte Ausführungsbeispiel eine ähnliche Rasthakenkonstruktion mit Rastnut und/oder Verdickung aufweisen.

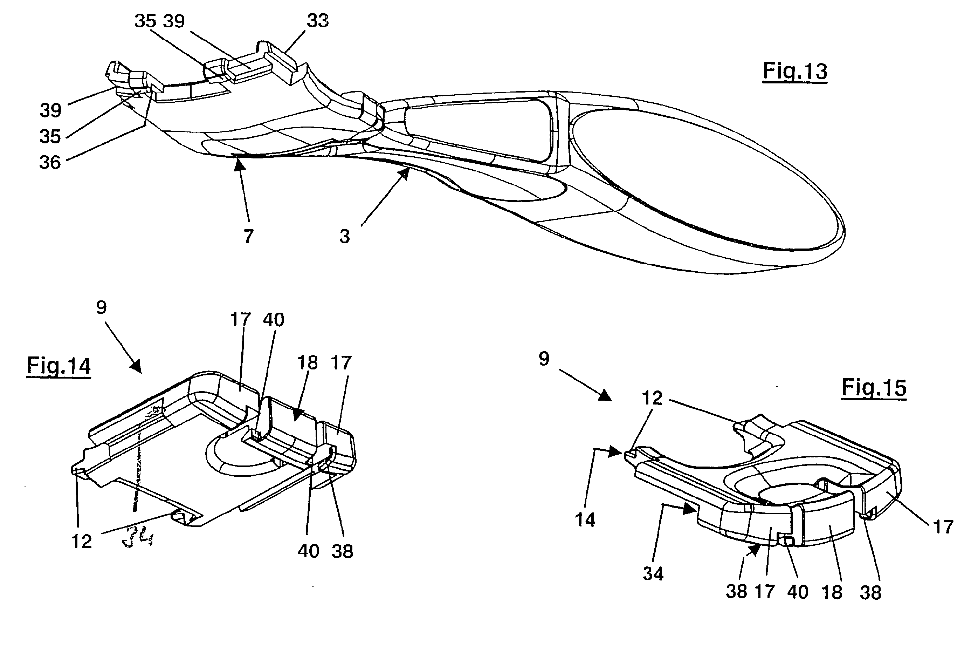

Anhand der Figuren 11 bis 15 wird nun ein drittes Ausführungsbeispiel des erfindungsgemäßen Hornhauthobels 1 beschrieben, wobei in den Figuren 11, 12 und 15 eine perspektivische Aufsicht und in den Figuren 13 und 14 eine perspektivische Untersicht des Hornhauthobels bez. einzelner Bauteile des Hornhauthobels gezeigt ist. Hierbei unterscheidet sich das dritte Ausführungsbeispiel von den vorherigen Ausführungsbeispielen im wesentlichen durch eine andere Ausbildung der Befestigungseinrichtung, die im Prinzip als eine Kombination der zuvor beschriebenen Befestigungsarten ausgelegt ist, d.h. nebst einigen konstruktiven Veränderungen vornehmlich eine Nut/Feder-Führung und eine Steckverbindung aufweist. Hierbei ist in dem freien Endabschnitt des Klingenkopfes 2 eine Nut/Feder-Führung vorgesehen, während das dem Handgriff 3 zugewandte Ende des Klingenkopfes 2 eine Steckverbindung aufweist.

Wie in Fig. 12 mit dem dort dargestellten unteren Halterungsteil 7 und in Fig. 14 und 15 mit dem oberen Halterungsteil 9 ersichtlich, weist das obere Halterungsteil 9 zwei sich in Funktionsrichtung X erstreckende Steckvorsprünge 12 auf, die unter zwei Haltevorsprünge 13 des unteren Halterungsteiles 7 jeweils gegen einen Anschlag 14 führbar sind. Hierbei sind die zueinander gewandten Seitenflächen der Vorsprünge 12, 13 nach dem Prinzip der schiefen Ebene aneinander abgleitbar angeordnet. Zweckmäßigerweise weisen die Haltevorsprünge 13 eine zu den Steckvorsprüngen 12 angepasste Beabstandung zum Boden der Aufnahme 8 auf, damit die in die Aufnahme 8 eingelegte Klinge 5 mit den Ecken Ihrer Schneide 6 unter die Haltevorsprünge 13 geführt werden kann. Hierdurch werden die gefährlichen Ecken der Schneide 6 abgedeckt und die Klinge 5 ferner lagestabil gehalten.

Abweichend von dem zweiten Ausführungsbeispiel sind, wie in Fig. 12 und 13 dargestellt, die seitlichen Führungsschiene 33 an dem freien Ende des unteren Halterungsteiles 7 angeordnet und erstrecken sich in ihrer Längsrichtung lediglich bis zur vorgesehenen Einlegeaussparung 21 zum Einlegen der Klinge 5 in die Aufnahme 8. Dementsprechend ist für das obere Halterungsteil 2 in Fig. 14 und 15 infolge von Überdeckung nicht sichtbare Führungsnuten 34 vorgesehen, die in ihrem Querschnitt und ihrer Länge den Führungsschienen 33 angepasst sind. Der Querschnitt der Führungsschiene 33 sowie der Führungsnuten 34 weist eine übliche schwalbenschwanzähnliche Form auf, mit deren Hilfe ein senkrecht zur Verschiebungsrichtung erfolgendes Verschieben verhindert wird. Hierbei ist zur Vereinfachung des Gegenstandes die Schwalbenschwanzform lediglich mit einer Hinterschneidung ausgeführt, wobei sich die Führungsschiene 33 in ihrer Quererstreckung schräg nach unten erstrecken.

Zur Verbindung beider Halterungsteile 7, 9 kann das obere Halterungsteil 9 von oben über das untere Halterungsteil 7 nur so weit über das freie Ende des unteren Halterungsteiles 7 überstehend auf die Aufnahme 8 aufgelegt werden, dass die Führungsschienen 33 des unteren Halterungsteiles 7 vor den Anfang der Führungsnut 34 positioniert sind und das obere Halterungsteil 9 mit seinen Führungsnuten 34 über die Führungsschienen 33 des unteren Halterungsteiles schiebbar sind. Hierbei wird eine in der Aufnahme 8 eingelegte Klinge 5 unter Aufbau einer Biegespannung nach unten zum Aufnahmegrund hin gedrückt. Mit dem Einführen der Führungsschienen 33 in die entsprechenden Führungsnuten 34 in Funktionsrichtung X des Hornhauthobels 1 ist ein geführtes Verschieben des oberen Halterungsteiles 9 in Funktionsrichtung X möglich, sodass die Steckvorsprünge 12 des oberen Halterungsteiles 9 unter die Haltevorsprünge 13 des unteren Halterungsteiles 7 führbar sind, bis die Steckvorsprünge 12 gegen den Anschlag 14 anliegen.

Zur festeren Positionierung sind die Führungshaken 17, abweichend von dem ersten Ausführungsbeispiel, jeweils mit einem zusätzlichen, sich in Funktionsrichtung X erstreckenden Führungshakenvorsprung 38 ausgestattet, der in zusammengefügtem Zustand der Halterungsteile 7, 9 in eine entsprechend angepasste, an der Unterseite des unteren Halterungsteiles 7 angeordnete Führungshakenausnehmung 39 eingreift. Hierbei sind Führungshakenvorsprung 38 und Führungshakenausnehmung 39 bevorzugt so dimensioniert, dass der Führungshakenvorsprung 38 nicht nach unten über die Unterseite des unteren Halterungsteiles 7 vorsteht.

Der vorgesehene Rasthaken 18 ist, wie in den vorhergehenden Ausführungsbeispielen, zwischen und parallel beabstandet zu den Führungshaken 17 gleich einer Blattfeder angeordnet und übergreift stirnseitig über das freie Ende des unteren Halterungsteiles 7. Hierdurch kann das Rasthaken 18 in einem bestimmten Umfang um eine Schwenkachse senkrecht zur Funktionsrichtung X hin und her bewegt werden. Abweichend von den vorhergehenden Beispielen weist der Rasthaken 18 seitlich angeordnete Rastflügel 40 auf, die zur Verrastung jeweils über einen an der Unterseite des unteren Halterungsteiles 7 angeordneten Rastvorsprunges 35 in eine in Funktionsrichtung X danach angeordnete als Tasche ausgebildete Rastnut 36 führbar sind. Zur Entrastung kann der Rasthaken 18 senkrecht zur Funktionsrichtung X nach unten gedrückt werden, sodass die Rastflügel 40 durch Verschieben des oberen Halterungsteiles 9 entgegen der Funktionsrichtung X über die jeweiligen Rastvorsprünge 35 führbar sind. Wie insbesondere in Fig. 11 und Fig. 13 ersichtlich ist die Befestigungseinrichtung mit den zur Unterseite des unteren Halterungsteiles 7 geführten Führungshaken 17 und Rasthaken 18 so dimensioniert, dass der Hornhauthobel eine glatte, durchgehende und somit nicht den Einsatz des Hornhauthobels 1 störende Unterseite aufweist.

Die hier dargestellten Ausführungsbeispiele des Hornhauthobels 1 sind mit den beiden Bauteilen, dem unteren mit dem Handgriff 3 verbundenen unteren Halterungsteil 7 und dem oberen Halterungsteil 9, als zweistückiges Spritzgussteil aus Kunststoff hergestellt. Denkbar ist auch die Herstellung des Hornhauthobels als einstückiges Spritzgussteil, indem das untere und das obere Halterungsteil über ein angegossenes Gelenk, zum Beispiel über ein Foliengelenk, einstückig miteinander verbunden sind.

### Bezugszeichenliste

- 1: Hornhauthobel
- 2: Klingenkopf
- 3: Handgriff
- 4: Halterung
- 5: Klinge
- 6: Schneide
- 7: unteres Halterungsteil
- 8: Aufnahme
- 9: oberes Halterungsteil
- 10: Ersatzschneide
- 11: Klingenöffnung
- 12: Steckvorsprung
- 13: Haltevorsprung
- 14: Anschlag
- 15: Seitenfläche
- 16: Führungssockel
- 17: Führungshaken
- 18: Rasthaken
- 19: Aussparung
- 20: Andrückmulde
- 21: Einlegaussparung
- 22: Durchgang
- 23: Abgleitschräge
- 24: Anschlussabschnitt
- 25: Fingerabschnitt
- 26: Handballenabschnitt
- 27: Steg
- 28: Sichtöffnung
- 29: Fingermulde
- 30: Griffpolster
- 31: Seitenfläche
- 32: Führungsvorsprung
- 33: Führungsschiene
- 34: Führungsnut
- 35: Rastvorsprung
- 36: Rastnut
- 37: Verdickung
- 38: Führungshakenvorsprung
- 39: Führungshakenausnehmung
- 40: Rastflügel
- a: Abstand
- b: Abstand
- X: Funktionsrichtung
- K: Kraftrichtung

## Patentansprüche

1. Hornhauthobel mit einem Klingenkopf (2) und einem Handgriff (3), wobei der Hornhauthobel (1) eine in Arbeitslage zur Körperoberfläche eines Benutzers gewandte Unterseite sowie eine von der Unterseite abgewandte Oberseite aufweist, in dem Klingenkopf (2) eine Halterung (4) mit einem unteren Halterungsteil (7) und einem oberen Halterungsteil (9) vorgesehen ist, in welcher eine Klinge (5) so halterbar ist, dass ihre zum Einsatz vorgesehene Schneide (6) zum Abhobeln von Hornhaut an der Unterseite des Hornhauthobels (1) angeordnet ist, das untere Halterungsteil (7) mit dem Handgriff (3) fest verbunden ist und eine nach oben weisenden Aufnahme (8) für die Klinge (5) aufweist und das obere Halterungsteil (9) die Klinge in der Aufnahme (8) mit Hilfe einer Befestigungseinrichtung festhält, **dadurch gekennzeichnet, dass** die Befestigungseinrichtung Verrastungsmittel zur Verrastung des oberen Halterungsteils (9) mit dem unteren Halterungsteil (7) aufweist.

2. Hornhauthobel nach Anspruch 1, **dadurch gekennzeichnet, dass** die Unterseite des Klingenkopfes (2) übergangslos von dem unteren Halterungsteil (7) in die Unterseite des Handgriffs (3) übergeht.

3. Hornhauthobel nach Anspruch 1 oder 2, **dadurch gekennzeichnet, dass** das untere Halterungsteil (7) die gesamte Unterseite der gehalterten Klinge (5) bis auf einen mittleren Abschnitt der zum Einsatz vorgesehenen Schneide (6) abdeckt.

4. Hornhauthobel nach einem der Ansprüche 1 bis 3, **dadurch gekennzeichnet, dass** der Klingenkopf (2) die Oberseite der gehalterten Klinge (5) bis auf einen mittleren Abschnitt der zum Einsatz vorgesehenen Schneide (6) im wesentlichen abdeckt.

5. Hornhauthobel nach einem der Ansprüche 1 bis 4, **dadurch gekennzeichnet, dass** die Unterseite des oberen Halterungsteils (9) und die Oberseite des unteren Halterungsteils (7) eine etwa parallel zueinander verlaufende, gebogene Form aufweisen, deren Biegeachse in einer Funktionsrichtung (X) des Hornhauthobels (1) verläuft, sodass die gehalterte Klinge unter Biegespannung zwischen den Halterungsteilen (7, 9) angeordnet ist.

6. Hornhauthobel nach einem der Ansprüche 1 bis 5, **dadurch gekennzeichnet, dass** der das untere Halterungsteil (7) eine Klingenöffnung (11) aufweist, durch die die Schneide (6) der Klinge (5) so führbar ist, dass zumindest ein mittlerer Längsabschnitt der Schneide (6) den Rand der Klingenöffnung (11) an der Unterseite des unteren Halterungsteils (7) senkrecht zur Funktionsrichtung (X)um einen kleinen Betrag überragt.

7. Hornhauthobel nach Anspruch 6, **dadurch gekennzeichnet, dass** die Unterseiten beider Halterungsteile (7, 9) gebogen sind, wobei der Biegeradius der Unterseite des unteren Halterungsteils (7) zumindest im Bereich der Klingenöffnung (11) geringer als der Biegeradius der Unterseite des oberen Halterungsteils (4) ist.

8. Hornhauthobel nach einem der Ansprüche 1 bis 7, **dadurch gekennzeichnet, dass** die Befestigungseinrichtung eine Führungsvorrichtung zum Einlegen der Klinge (5) in die Aufnahme (8) sowie zum Ineinanderführen beider Halterungsteile (7, 9) aufweist.

9. Hornhauthobel nach Anspruch 8, **dadurch gekennzeichnet, dass** die Führungsvorrichtung Führungssockel (16) aufweist, die an den Ecken der Aufnahme (8) angeordnet sind und sich nach oben hin erstrecken, wobei die gehalterte Klinge (5) sowie das obere Halterungsteil (9) seitlich an den Führungssockeln (16) anliegen.

10. Hornhauthobel nach einem der Ansprüche 1 bis 9, **dadurch gekennzeichnet, dass** die Befestigungseinrichtung eine Nut/Feder-Führung aufweist, durch die das obere Halterungsteil (9) über das untere Halterungsteil (7) führbar ist.

11. Hornhauthobel nach Anspruch 10, **dadurch gekennzeichnet, dass** die Nut/Feder-Führung eine Führungsschiene (33) und eine Führungsnut (34) mit schwalbenschwanzartigem Querschnitt aufweist.

12. Hornhauthobel nach einem der Ansprüche 1 bis 11, **dadurch gekennzeichnet, dass** an dem freien Ende des Klingenkopfes (2) und an der Oberseite des oberen Halterungsteils (9) eine Andrückmulde (20) vorgesehen ist.

13. Hornhauthobel nach einem der Ansprüche 1 bis 12, **dadurch gekennzeichnet, dass** in dem oberen Halterungsteil (9) einen Durchgang (22) zum Sammeln und Auswerfen abgehobelter Hornhautpartikel vorgesehen ist, wobei der Durchgang (22) in Arbeitslage über der Klingenöffnung (11) angeordnet ist.

14. Hornhauthobel nach einem der Ansprüche 1 bis 13, **dadurch gekennzeichnet, dass** der Handgriff (3) an seinem freien Ende ein Griffteil und an seinem zum Klingenkopf (2) gewandten Ende einen Anschlussabschnitt (24) aufweist, wobei in dem Anschlussabschnitt (24) eine von der Oberseite zur Unterseite führende Sichtöffnung (28) zur Sicht auf die zu bearbeitende Körperoberfläche eines Benutzers eingelassen ist.

15. Hornhauthobel nach Anspruch 14, **dadurch gekennzeichnet, dass** das Griffteil zur ergonomischen Anpassung an seinem freien Endbereich einen Handballenabschnitt (26) mit konvexen Griffpolstern (39) und/oder in seinem dem Anschlussabschnitt (24) zugewandten Ende einen Fingerabschnitt (25) mit einer Fingermulde (29) aufweist.

16. Hornhauthobel nach Anspruch 15, **dadurch gekennzeichnet, dass** der Fingerabschnitt (25) eine viereckige Querschnittsform mit allseitig vorgesehenen Fingermulden (29) aufweist.

17. Hornhauthobel nach einem der Ansprüche 1 bis 16, **dadurch gekennzeichnet, dass** der Hornhauthobel (1) aus Kunststoff hergestellt ist.

## Claims

1. Callous plane having a blade head (2) and a handle (3), where the callous plane (1) has an underside facing the body surface of a user when in working position, and an upper side facing away from said underside, and where a holding device (4) with a lower holding part (7) and an upper holding part (9) is provided in the blade head (2), in which a blade (5) can be held in such a manner that its cutting edge (6) intended for use to shave off callous skin is located on the underside of the callous plane (1), wherein the lower holding part (7) is permanently connected to the handle (3) and displays an upward-pointing seat (8) for the blade (5), and in that the upper holding part (9) holds the blade tightly in the seat (8) with the help of a fixing device **characterised in that** the fixing device displays snap-in means for snap-fitting the upper holding part (9) to the lower holding part (7).

2. Callous plane according to Claim 1, **characterised in that** the underside of the blade head (2) transitions smoothly from the lower holding part (7) into the underside of the handle (3).

3. Callous plane according to Claim 1 or 2, **characterised in that** the lower holding part (7) covers the entire underside of the held blade (5), apart from a middle section of the cutting edge (6) intended for use.

4. Callous plane according to one of Claims 1 to 3, **characterised in that** the blade head (2) essentially covers the upper side of the held blade (5), apart from a middle section of the cutting edge (6) intended for use.

5. Callous plane according to one of Claims 1 to 4, **characterised in that** the underside of the upper holding part (9) and the upper side of the lower holding part (7) display curved shapes running roughly parallel to each other, the neutral axis of which runs in a working direction (X) of the callous plane (1), such that the held blade is located between the holding parts (7, 9) under bending stress.

6. Callous plane according to one of Claims 1 to 5, **characterised in that** the lower holding part (7) displays a blade opening (11), through which the cutting edge (6) of the blade (5) can be passed in such a way that at least a middle longitudinal section of the cutting edge (6) projects beyond the edge of the blade opening (11) on the underside of the lower holding part (7) by a small distance perpendicular to the working direction (X).

7. Callous plane according to Claim 6, **characterised in that** the undersides of both holding parts (7, 9) are curved, where the bend radius of the underside of the lower holding part (7) is smaller than the bend radius of the underside of the upper holding part (9), at least in the area of the blade opening (11).

8. Callous plane according to one of Claims 1 to 7, **characterised in that** the fixing device displays a guide device for inserting the blade (5) into the seat (8), and for inserting the two holding parts (7, 9) into each other.

9. Callous plane according to Claim 8, **characterised in that** the guide device displays guide prongs (16) that are located at the corners of the seat (8) and extend upwards, where the held blade (5) and the upper holding part (9) laterally rest against the guide prongs (16).

10. Callous plane according to one of Claims 1 to 9, **characterised in that** the fixing device displays a tongue-and-groove guide, by means of which the upper holding part (9) can be guided over the lower holding part (7).

11. Callous plane according to Claim 10, **characterised in that** the tongue-and-groove guide displays a guide rail (33) and a guide groove (34) with a dovetail-type cross-section.

12. Callous plane according to one of Claims 1 to 11, **characterised in that** a pressure hollow (20) is provided on the free end of the blade head (2) and on the upper side of the upper holding part (9).

13. Callous plane according to one of Claims 1 to 12, **characterised in that** a through-passage (22) for collecting and ejecting shaved-off callous skin particles is provided in the upper holding part (9), where the through-passage (22) is located above the blade opening (11) when in working position.

14. Callous plane according to one of Claims 1 to 13, **characterised in that** the handle (3) displays a handle part at its free end and a connecting section (24) at its end facing the blade head (2), where an eyehole (28) leading from the upper side to the underside is let into the connecting section (24) in order to give a view of the body surface of a user to be treated.

15. Callous plane according to Claim 14, **characterised in that,** for ergonomic adaptation, the handle part displays a section for the ball of the thumb (26) with convex padded grips (30) in its free end area and/or a section for the fingers with a finger recess (29) on its end facing towards the connecting section (24).

16. Callous plane according to Claim 15, **characterised in that** the section for the fingers (25) displays a rectangular cross-sectional shape with finger recesses (29) provided on all sides.

17. Callous plane according to one of Claims 1 to 16, **characterised in that** the callous plane (1) is made of plastic.

## Revendications

1. Râpe pour callosités avec une tête porte-lame (2) et une poignée (3), la râpe pour callosités (1) présentant une face inférieure tournée vers la surface corporelle d'un utilisateur, ladite râpe étant en position d'utilisation, et une face supérieure, opposée à la face inférieure, un élément de retenue (4) avec une partie de retenue inférieure (7) et une partie de retenue supérieure (9) dans lequel une lame (5) peut être maintenue de sorte que son tranchant (6) prévu pour éliminer les callosités se trouve sur la face inférieure de la râpe pour callosités (1) étant prévu dans la tête porte-lame (2), la partie de retenue inférieure (7) étant reliée fixement à la poignée (3) et présentant une cavité (8) dirigée vers le haut pour la lame (5) et la partie de retenue supérieure (9) maintenant la lame dans la cavité (8) à l'aide d'un dispositif de fixation, **caractérisée en ce que** le dispositif de fixation présente des moyens d'enclenchement pour enclencher la partie de retenue supérieure (9) avec la partie de retenue inférieure (7).

2. Râpe pour callosités selon la revendication 1, **caractérisée en ce que** la face inférieure de la tête porte-lame (2) passe sans transition de la partie de retenue inférieure (7) à la face inférieure de la poignée (3).

3. Râpe pour callosités selon la revendication 1 ou 2, **caractérisée en ce que** la partie de retenue inférieure (7) couvre toute la face inférieure de la lame (5) maintenue, excepté une section centrale du tranchant (6) prévu pour utilisation.

4. Râpe pour callosités selon l'une quelconque des revendications 1 à 3, **caractérisée en ce que** la tête porte-lame (2) couvre essentiellement la face supérieure de la lame (5) maintenue, excepté une section centrale du tranchant (6) prévu pour utilisation.

5. Râpe pour callosités selon l'une quelconque des revendications 1 à 4, **caractérisée en ce que** la face inférieure de la partie de retenue supérieure (9) et la face supérieure de la partie de retenue inférieure (7) présentent une forme cintrée, à peu près parallèle l'une par rapport à l'autre, dont l'axe de pliage passe dans un sens de fonctionnement (X) de la râpe pour callosités (1), de sorte que la lame maintenue est disposée, sous contrainte de flexion, entre les parties de retenue (7, 9).

6. Râpe pour callosités selon l'une quelconque des revendications 1 à 5, **caractérisée en ce que** la partie de retenue inférieure (7) présente une ouverture de lame (11) à travers laquelle le tranchant (6) de la lame (5) peut être guidé, de sorte qu'au moins une section longitudinale centrale du tranchant (6) dépasse, d'un petit peu, le bord de l'ouverture de lame (11) au niveau de la face inférieure de la partie de retenue inférieure (7), et ce, perpendiculairement au sens de fonctionnement (X).

7. Râpe pour callosités selon la revendication 6, **caractérisée en ce que** les faces inférieures des deux parties de retenue (7, 9) sont pliées, sachant que le rayon de courbure de la face inférieure de la partie de retenue inférieure (7) est, au moins dans la zone de l'ouverture de lame (11), inférieur au rayon de courbure de la face inférieure de la partie de retenue supérieure (9).

8. Râpe pour callosités selon l'une quelconque des revendications 1 à 7, **caractérisée en ce que** le dispositif de fixation présente un dispositif de guidage pour placer la lame (5) dans la cavité (8) et pour guider l'une dans l'autre les deux parties de retenue (7, 9).

9. Râpe pour callosités selon la revendication 8, **caractérisée en ce que** le dispositif de guidage présente des socles de guidage (16) qui sont disposés au niveau des angles de la cavité (8) et qui s'étendent vers le haut, sachant que la lame (5) maintenue, ainsi que la partie de retenue supérieure (9) s'appliquent latéralement contre les socles de guidage (16).

10. Râpe pour callosités selon l'une quelconque des revendications 1 à 9, **caractérisée en ce que** le dispositif de guidage présente un guidage à rainure et languette qui permet de guider la partie de retenue supérieure (9) sur la partie de retenue inférieure (7).

11. Râpe pour callosités selon la revendication 10, **caractérisée en ce que** le guidage à rainure et languette présente une glissière de guidage (33) et une rainure de guidage (34) avec une section du type queue d'aronde.

12. Râpe pour callosités selon l'une quelconque des revendications 1 à 11, **caractérisée en ce qu'**il est prévu un creux de pression (20) au niveau de l'extrémité libre de la tête porte-lame (2) et au niveau de la face supérieure de la partie de retenue supérieure (9).

13. Râpe pour callosités selon l'une quelconque des revendications 1 à 12, **caractérisée en ce qu'**il est prévu, dans la partie de retenue supérieure (9), un passage (22) pour recueillir et éjecter des particules de callosités râpées, sachant que le passage (22) est disposé, en position d'utilisation, au-dessus de l'ouverture de lame (11).

14. Râpe pour callosités selon l'une quelconque des revendications 1 à 13, **caractérisée en ce que** la poignée (3) présente, au niveau de son extrémité libre, une partie de serrage et au niveau de son extrémité tournée vers la tête porte-lame (2), une section de raccordement (24), sachant qu'une ouverture de visualisation (28) menant de la face supérieure à la face inférieure pour voir sur la surface corporelle à traiter d'un utilisateur est insérée dans la section de raccordement (24).

15. Râpe pour callosités selon la revendication 14, **caractérisée en ce que,** pour une adaptation ergonomique, la partie de serrage présente au niveau de sa zone terminale libre une section pour la paume de la main (26) avec des garnitures intérieures de serrage (39) convexes et/ou dans son extrémité tournée vers la section de raccordement (24) une section pour les doigts (25) avec un creux pour les doigts (29).

16. Râpe pour callosités selon la revendication 15, **caractérisée en ce que** la section pour les doigts (25) présente une forme de section transversale rectangulaire avec des creux pour les doigts (29) prévus de tous côtés.

17. Râpe pour callosités selon l'une quelconque des revendications 1 à 16, **caractérisée en ce que** la râpe pour callosités (1) est fabriquée en matière plastique.
